# DEMANDE DE BREVET EUROPEEN

(11) **EP 1 103 249 A1**
(43) Date de publication de la demande: **30.05.2001**
(21) Numéro de dépôt: 00403047.4
(22) Date de dépôt: 02.11.2000
(51) Int. Cl.: A61K 7/32

(54) **Composition cosmétique déodorante anhydre**

(30) Priorité: 25.11.1999 FR 9914851
(71) Demandeur: L'OREAL, 75008 Paris (FR)
(72) Inventeur: Pataut, Françoise, 75017 Paris (FR); Aubert, Lionnel, 95270 Asnieres sur Oise (FR)
(74) Mandataire: Miszputen, Laurent

(57) **Abrégé**

L'invention concerne une composition cosmétique déodorante anhydre comprenant au moins un actif déodorant et au moins un copolymère bloc choisi dans le groupe comprenant les copolymères di-, tri-, multi-, ou radial blocs et leurs mélanges, lesdits copolymères blocs étant composés de segments de type styrène monomère et de segments de type monomère ou comonomère thermoplastique, caractérisée par le fait qu'elle contient en outre au moins une substance absorbant le gras et au moins une huile synthétique.

## Description

La présente invention concerne une composition cosmétique déodorante anhydre comprenant au moins un actif déodorant et au moins un copolymère bloc, caractérisée par le fait qu'elle contient en outre au moins une substance absorbant le gras et au moins une huile synthétique.

Bien des types différents de compositions déodorantes non aqueuses (anhydres) ont été décrits dans la littérature et sont apparus sur le marché sous des formes telles que des gels, des sticks, des crèmes, des roll-ons ou des aérosols. Parmi ces formes, les gels, les sticks et les crèmes sont constitués d'une substance de base liquide qui est solidifiée par un agent de consistance et ce sont ces formes semi-solides ou solides qui forment un des objets de la présente invention.

Généralement, les dites formes comprennent une solution de l'ingrédient actif dans un solvant convenablement choisi, une suspension de l'ingrédient actif dans un milieu non-solvant, ou une dispersion ou émulsion (i)dans la phase continue de laquelle une solution de l'ingrédient actif est dispersée ou (ii)pour laquelle l'actif solubilisé constitue la phase continue.

Ainsi, on recherche des compositions cosmétiques déodorantes toujours plus performantes en terme de stabilité, d'homogénité, et de bonnes propriétés rhéologiques à l'application. Lesdites compositions ne doivent en outre pas laisser de résidu visible (blanc) sur la peau à l'application ou après séchage de la composition après l'application, tout en laissant une peau douce au toucher.

Par ailleurs, on a antérieurement préconisé dans la demande de brevet WO 94/12190 d'utiliser dans les sticks déodorants des agents gélifiants constitués de copolymères blocs styrène/élastomère dans des huiles minérales. Cependant, de tels sticks laissent un résidu gras sur la peau tout à fait indésirable et ne présentent pas tous les avantages recherchés et cités ci-dessus.

Après de nombreuses recherches menées sur la question, la Demanderesse a maintenant découvert, de façon inattendue et surprenante, qu'en introduisant dans une composition cosmétique déodorante comprenant au moins un actif déodorant et un copolymère bloc à base de styrène et d'un monomère ou comonomère thermoplastique, une quantité efficace d'une substance absorbant le gras et d'une huile synthétique, on pouvait notablement améliorer les compositions et obtenir tout à la fois les propriétés recherchées, c'est à dire des compositions stables et homogènes, de viscosité en accord avec les formes de présentation désirées (sticks, gels ou crèmes), qui n'exudent pas, s'étalent très bien sur la peau, sans laisser de résidu visible blanc et/ou gras à l'application ou après séchage après l'application.

Cette découverte est à la base de la présente invention.

La présente invention a ainsi pour premier objet une composition cosmétique déodorante anhydre comprenant au moins un actif déodorant et au moins un copolymère bloc choisi dans le groupe comprenant les copolymères di-, tri-, multi- ou radial blocs et leurs mélanges, lesdits copolymères blocs étant composés de segments de type styrène monomère et de segments de type monomère ou comonomère thermoplastique, caractérisée par le fait qu'elle contient en outre au moins une substance absorbant le gras et au moins une huile synthétique.

Elle a plus particulièrement pour objet une composition cosmétique déodorante anhydre comprenant 0,1 à 40% en poids d'au moins un actif déodorant, 0,1 à 7% en poids d'au moins un copolymère di-, tri-, multi- ou radial bloc composé de segments de type styrène monomère et de segments de type monomère ou comonomère thermoplastique, 0,1 à 20% en poids d'au moins une substance absorbant le gras et 5 à 90% en poids d'au moins une huile synthétique.

Au sens de la présente invention, on entend par actif déodorant toute substance capable de réduire le flux sudoral et/ou masquer, améliorer ou réduire l'odeur désagréable résultant de la décomposition de la sueur humaine par des bactéries.

Il s'agit par exemple d'un composé anti-transpirant tel que, et sans être limitatif, les sels d'aluminium et/ou de zirconium, par exemple un chlorhydrate d'aluminium, un hydroxychlorure d'aluminium ou un chlorhydrate d'aluminium et de zirconium, les sels d'alun, d'un agent bactériostatique, d'un agent bactéricide tel que le 2,4,4'-trichloro-2'-hydroxydiphényléther et le 3,7,11-triméthyldodéca-2,5,10-triénol, et différents sels de zinc, d'un agent absorbant les odeurs tel que le bicarbonate de sodium ou le pidolate de zinc (pyrrolidone carboxylate de zinc).
Le 3,7,11-triméthyldodéca-2,5,10-triénol est par exemple vendu sous la dénomination FARNESOL® par la société DRAGOCO et le 2,4,4'-trichloro-2'-hydroxydiphényléther sous la dénomination IRGACARE® MP par la société CIBA-GEIGY.

Parmi les sels d'aluminium, on peut citer plus particulièrement l'hydroxychlorure d'aluminium commercialisé par la société REHEIS sous la dénomination REACH 301 ou par la société GUILINI CHEMIE sous la dénomination ALOXICOLL PF 40. Des sels d'aluminium et de zirconium sont par exemple celui commercialisé par la société REHEIS sous la dénomination REACH A2P-908-SUF .
Des complexes d'hydroxychlorure de zirconium et d'hydroxychlorure d'aluminium et de glycine, communément connus sous l'appellation "ZAG complexes", sont également utilisables selon l'invention.

Le ou les copolymères di-, tri-, multi- ou radial blocs composés de segments de type styrène monomère et de segments de type monomère ou comonomère thermoplastique utilisables selon l'invention sont décrits dans le brevet US-5 221 534 faisant partie intégrante de la présente demande.
Parmi ces copolymères blocs, on préfère utiliser notamment ceux pour lesquels le monomère ou comonomère thermoplastique désigne éthylène/alkylène en C₃-C₄, et plus particulièrement encore un copolymère hydrogéné à blocs styrène et à blocs éthylène/alkylène en C₃-C₄.
On utilise plus avantageusement encore selon l'invention, un mélange de copolymère hydrogéné à blocs butylène/éthylène et à blocs styrène et de copolymère hydrogéné à blocs éthylène/propylène et à blocs styrène, dans de l'huile minérale, et notamment un mélange de 1 à 20% en poids de copolymère hydrogéné à blocs butylène/éthylène et à blocs styrène et de copolymère hydrogéné à blocs éthylène/propylène et à blocs styrène dans 80 à 99% en poids d'huile minérale.
De tels mélanges sont par exemple vendus par la société PENRECO sous les dénominations commerciales VERSAGEL M200 et VERSAGEL M750 ou par la société AIGLON sous les dénominations commerciales TRANSGEL ou SYNGEL ( 90% d'huile de paraffine, 5% de copolymère hydrogéné butylène/éthylène/styrène, 5% de copolymère hydrogéné éthylène/propylène/styrène).

Les substances absorbant le gras utilisables dans la composition selon l'invention peuvent être choisies parmi des particules creuses d'une granulométrie convenable telles que décrites ci-après, ou bien encore le talc, l'amidon et ses dérivés, l'argile, la silice, les polyoléfines, le polystyrène, le téflon, et leurs mélanges.
Parmi les dérivés d'amidon on peut citer par exemple l'amidon de maïs estérifié vendu sous la dénomination commerciale DRYFLO PLUS par la société NATIONAL STARCH, le phospate de diamidon de pomme de terre hydroxypropylé non prégélatinisé vendu sous la dénomination commerciale FARINEX VA-100 par la société AVEBE.
Parmi les silices on peut citer comme exemple des microsphères de silice poreuse vendues sous la dénomination commerciale SILICA BEAD SB-700 par la société MIYOSHI, ou la silice pyrogénée hydrophobe vendue sous la dénomination commerciale AEROSIL R 972 par la société DEGUSSA. Comme argile, on peut citer l'argile verte vendue sous la dénomination commerciale GAMMA 2 KGY par la société BENTOFRANCE.
Comme polyéthylène, on peut citer par exemple la poudre de polyéthylène vendue sous la dénomination commerciale ACUMIST B6 par la société ALLIED CHEMICAL.

Les particules creuses qui peuvent être utilisées dans la composition déodorante selon l'invention présentent de façon avantageuse une granulométrie moyenne allant de 5 µm à 200 µm et de préférence de 10 µm à 100 µm et mieux de 15 µm à 60 µm.

Les particules sont avantageusement réalisées en matériaux thermoplastiques comme les polyamides tels que le nylon, les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'a-méthyl-styrène ou le styrène.
Comme particules de nylon, on peut utiliser les particules d' « Orgasol » vendues par la société Atochem. Ces particules sont des sphères pleines, poreuses, de diamètre allant de 5 µm à 60 µm.

De préférence, les particules sont des particules creuses d'un copolymère expansé, de chlorure de vinylidène et d'acrylonitrile, ou d'un terpolymère expansé de chlorure de vinylidène, d'acrylonitrile et de méthacrylate. On peut par exemple utiliser un terpolymère contenant : de 0 % à 60% de motifs dérivés du chlorure de vinylidène, de 20% à 90% de motifs dérivés d'acrylonitrile et de 0% à 50% motifs dérivés d'un monomère acrylique ou styrénique, la somme des pourcentages (en poids) étant égale à 100. Ces particules peuvent être sèches ou hydratées et sont, par exemple, celles vendues sous la marque EXPANCEL par la société Nobel Casco, et en particulier sous les références 551 DE (granulométrie d'environ 50 µm et masse volumique d'environ 35 kg/m³), 551 DE 12 (granulométrie d'environ 12 µm et masse volumique d'environ 40 kg/m³), 551 DE 20 (granulométrie d'environ 30 µm et masse volumique d'environ 65 kg/m³), 551 DE 50 (granulométrie d'environ 40 µm), 461 DE 50 et 642 WE 50 de 50 µm de granulométrie environ, 551 DE 80 (granulométrie de 80 µm environ).

On peut aussi utiliser des particules de ce même terpolymère ayant une granulométrie d'environ 18 µm et une masse volumique d'environ 60 à 90 kg/m³, ou encore de granulométrie d'environ 34 µm et de masse volumique d'environ 20 kg/m³.

On peut encore utiliser des particules de copolymère non expansé de chlorure de vinylidène et d'acrylonitrile, ou de terpolymère non expansé de chlorure de vinylidène, d'acrylonitrile et de méthacrylate, comme celles vendues sous la marque EXPANCEL avec la référence 551 DU 10 (granulométrie d'environ 10 µm), ou 461 DU 15 (granulométrie d'environ 15 µm).

Comme autre particules creuses polymériques utilisables selon l'invention, on peut encore citer les polymères et les copolymères obtenus à partir des esters (acétate ou lactate de vinyle), ou acides (itaconique, citraconique, maléique, fumarique) [voir à cet effet le document JP-A-2-112304].

Selon une autre caractéristique avantageuse de l'invention, les particules se présentent sous forme de billes. Il est toutefois possible d'utiliser des particules ayant la forme de fibres ou d'aiguilles.

Les particules vendues sous la marque Expancel, selon l'invention, peuvent être obtenues, par exemple, selon les procédés des brevets et demandes de brevet EP-56 219, EP-348 372, EP-486 080, EP-320 473, EP-112 807, US-3 615 972. La cavité interne des particules contient en principe un gaz qui peut être de l'air, de l'azote ou un hydrocarbure comme l'isobutane ou l'isopentane.

D'autres objets de la présente invention concernent des compositions cosmétiques déodorantes anhydres comprenant :
- (a) 0,1 à 40% en poids d'au moins un actif déodorant,
- (b) 0,1 à 7% en poids d'au moins un copolymère di-, tri-, multi- ou radial bloc composé de segments de type styrène monomère et de segments de type monomère ou comonomère thermoplastique,
- (c) 0,1 à 10% en poids de particules creuses définies ci-avant et de préférence 0,1 à 5% en poids de particules creuses d'un terpolymère expansé de chlorure de vinylidène, d'acrylonitrile et de méthacrylate, et,
- (d) 5 à 90% en poids d'au moins une huile synthétique.

Les huiles synthétiques utilisables selon la présente invention sont de préférence choisies parmi les isoparaffines ou leurs mélanges de formule (I) suivante : 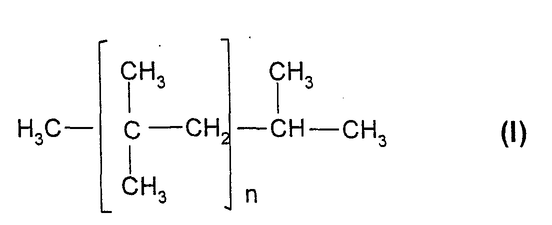 pour laquelle n ≥ 2 et de préférence compris entre 2 et 40, dont on préfère avantageusement celles de formule (I) pour laquelle n =3 (isohexadécane) et n=2 (isododécane).
Parmi elles on peut citer celles dans lesquelles n est égal à 2, 3, 4, 16, ou 38 et en particulier les produits vendus sous les dénominations PERMETHYL 99A, 101A, 102A, 104A, 106A par la société PREPERSE INC, ou le produit ARLAMOL HD vendu par la société ICI, correspondant à la formule (I) dans laquelle n est égal à 3.

Dans la composition déodorante selon la présente invention, l'actif déodorant (au sens l'invention) est généralement présent dans des concentrations pondérales (ramenées à l'ensemble de la composition) allant environ de 0,1 à 40%, de préférence allant environ de 0,5 à 25%, le ou les copolymères blocs sont généralement présents dans des concentrations pondérales allant environ de 0,1 à 7 % , de préférence allant environ de 2 à 5%, la substance absorbant le gras est généralement présente dans des concentrations pondérales allant environ de 0,1 à 20% , de préférence allant environ de 0,5 à 10%, et l'huile synthétique est généralement présente dans des concentrations pondérales allant environ de 5 à 90% , de préférence allant environ de 20 à 60%.

La composition selon l'invention peut encore comprendre un ou des agents de consistance tels que notamment la cire de paraffine, l'alcool stéarylique, des cires d'origine naturelle comme les cires microcristallines, la cérésine, l'ozokérite, la cire de candellila, la cire de carnauba, l'huile de ricin hydrogénée, l'huile de palme hydrogénée, l'huile de coco hydrogénée, des cires polyéthyléniques telles que celles décrites dans le brevet français 2 776 187 faisant partie intégrante de la demande, et encore plus particulièrement des mélanges de cires naturelles et de cires polyéthyléniques ayant toutes un point de fusion supérieur à 80 °C tels que ceux décrits dans ledit brevet cité.

Lesdits agents de consistance sont présents dans la composition selon l'invention en une quantité suffisante pour conférer à la composition la forme désirée de gel, de crème ou de stick.

La composition selon l'invention peut encore contenir d'autres ingrédients bien connus dans le domaine des produits cosmétiques déodorants, dont on peut citer par exemple, des agents apaisants, des parfums, des conservateurs, des agents antioxydants, des séquestrants, des agents de suspension tels que les bentonites et les hectorites, des émollients tels que les esters d'acides gras comme le myristate ou le palmitate d'isopropyle.
La composition selon l'invention peut encore être pressurisée et être conditionnée dans un aérosol; elle contient alors les propulseurs généralement utilisés dans ce type de produits et bien connus de l'homme de l'art, comme par exemple les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT.
On peut également utiliser en tant qu'agent propulseur le gaz carbonique, le protoxyde d'azote, l'azote ou l'air comprimé.
L'agent propulseur représente alors de préférence de 20 à 85% en poids du poids total de la composition.

Bien entendu, l'homme de l'art veillera à choisir le ou les éventuels composés complémentaires mentionnés ci-avant, de manière telle que les propriétés avantageuses attachées intrinsèquement à la composition déodorante selon l'invention ne soient pas, ou substantiellement pas, altérées par la ou les adjonctions envisagées.

D'autres objets de la présente invention concernent l'utilisation cosmétique d'une composition telle que décrite ci-avant, pour réduire le flux sudoral et/ou masquer, améliorer ou réduire l'odeur désagréable résultant de la décomposition de la sueur humaine par des bactéries et un procédé pour traiter les odeurs axillaires humaines, consistant à appliquer sur la surface axillaire une quantité efficace d'une composition telle que décrite ci-avant.

Des exemples concrets, mais nullement limitatifs, illustrant l'invention vont maintenant être donnés.

### EXEMPLE 1 :

On a préparé une crème antitranspirante anhydre de composition suivante : (quantités exprimées en grammes)

| | |
|---|---|
| Versagel M 200 vendu par PENRECO | 25 |
| Hydroxychlorure d'aluminium anhydre micronisé | 25 |
| Phosphate de diamidon de pomme de terre hydroxypropylé (Farinex VA-100 de AVEBE) | 5 |
| Microsphères expansées de copolymère chlorure de vinylidène/acrylonitrile/méthacrylate de méthyle contenant de l'isobutane vendu sous la marque Expancel 551 DE 20 par la société Nobel Casco: | 1 |
| Isohexadécane | 10 |
| Isododécane | 28,95 |
| Ditertio-butyl-4-hydroxytoluène | 0,05 |
| Huile de ricin hydrogénée | 5 |

Dans une cuve de fabrication, on a introduit l'huile ricin hydrogénée puis on a chauffé à 95-100°C sous agitation.
On y a ensuite introduit un prémélange de Versagel M200, d'isohexadécane et d' isododécane, puis le ditertio-butyl-4-hydroxytoluène. Après fusion complète et homogénéisation, on a refroidi à 80°C. Puis on a ajouté l'hydroxychlorure d'aluminium anhydre micronisé, le phosphate de diamidon et l'Expancel. On a turbiné sous agitation forte puis diminué la température jusqu'à 50°C.

### EXEMPLE 2 :

On a préparé un stick antitranspirant anhydre de composition suivante : (quantités exprimées en grammes)

| | |
|---|---|
| Versagel M 200 vendu par PENRECO | 25 |
| Hydroxychlorure d'aluminium anhydre micronisé | 25 |
| Phosphate de diamidon de pomme de terre hydroxypropylé (Farinex VA-100 deAVEBE) | 5 |
| Microsphères expansées de copolymère chlorure de vinylidène/acrylonitrile/méthacrylate de méthyle contenant de l'isobutane vendu sous la marque Expancel 551 DE 20 par la société Nobel Casco | 1 |
| Isohexadécane | 10 |
| Isododécane | 13,95 |
| Ditertio-butyl-4-hydroxytoluène | 0,05 |
| Huile de ricin hydrogénée | 5 |
| Alcool stéarylique | 15 |

Dans une cuve de fabrication, on a introduit l'huile ricin hydrogénée et l'alcool stéarylique, puis on a chauffé à 95-100°C sous agitation.
On y a ensuite introduit un prémélange de Versagel M200, d'isohexadécane et d' isododécane, puis le ditertio-butyl-4-hydroxytoluène. Après fusion complète et homogénéisation, on a refroidi à 80°C. Puis on a ajouté l'hydroxychlorure d'aluminium anhydre micronisé, le phosphate de diamidon et l'Expancel. On a turbiné sous agitation forte puis diminué la température jusqu'à 65°C.

### EXEMPLE 3 :

On a préparé un aérosol antitranspirant anhydre de composition suivante : (quantités exprimées en grammes)

| | |
|---|---|
| JUS | |
| Versagel M 200 vendu par PENRECO | 5 |
| Isohexadécane | 6 |
| Isododécane | 5 |
| Hydroxychlorure d'aluminium anhydre micronisé | 3 |
| Microsphères expansées de copolymère chlorure de vinylidène/acrylonitrile/méthacrylate de méthyle contenant de l'isobutane vendu sous la marque Expancel 551 DE 20 par la société Nobel Casco | 0,5 |
| Bentonite (Bentone 38V de RHEOX) | 0,5 |
| PROPULSEUR | |
| lsobutane | 80 |

## Revendications

1. Composition cosmétique déodorante anhydre comprenant au moins un actif déodorant et au moins un copolymère bloc choisi dans le groupe comprenant les copolymères di-, tri-, multi-, ou radial blocs et leurs mélanges, lesdits copolymères blocs étant composés de segments de type styrène monomère et de segments de type monomère ou comonomère thermoplastique, caractérisée par le fait qu'elle contient en outre au moins une substance absorbant le gras et au moins une huile synthétique.

2. Composition cosmétique déodorante anhydre comprenant :
- (a) 0,1 à 40% % en poids d'au moins un actif déodorant,
- (b) 0,1 à 7% en poids d'au moins un copolymère di-, tri-, multi- ou radial bloc composé de segments de type styrène monomère et de segments de type monomère ou comonomère thermoplastique,
- (c) 0,1 à 20% en poids d'au moins une substance absorbant le gras et,
- (d) 5 à 90% en poids d'au moins une huile synthétique.

3. Composition selon les revendications 1 ou 2, caractérisée par le fait que la substance absorbant le gras est choisie parmi des particules creuses présentant une granulométrie moyenne allant de 1 µm à 300 µm.

4. Composition selon la revendication 3, caractérisée par le fait que les particules creuses présentent une granulométrie moyenne allant de 1 µm à 150 µm.

5. Composition selon la revendication 4, caractérisée par le fait que les particules creuses présentent une granulométrie moyenne allant de 10 µm à 100 µm.

6. Composition selon l'une quelconque des revendications 3 à 5, caractérisée par le fait que les particules creuses sont formées d'un matériau thermoplastique.

7. Composition selon l'une quelconque des revendications 3 à 6, caractérisée par le fait que les particules creuses sont réalisées en un matériau choisi parmi le nylon, les polymères et copolymères de polychlorure de vinylidène, d'acrylonitrile et/ou d'un monomère acrylique ou styrénique.

8. Composition selon l'une quelconque des revendications 3 à 7, caractérisée par le fait que les particules creuses sont réalisées en un matériau choisi parmi les polymères et copolymères de polychlorure de vinylidène, d'acrylonitrile et/ou d'un monomère acrylique ou styrénique, expansés.

9. Composition cosmétique déodorante anhydre comprenant :
- (a) 0,1 à 40% en poids d'au moins un actif déodorant,
- (b) 0,1 à 7% en poids d'au moins un copolymère di-, tri-, multi- ou radial bloc composé de segments de type styrène monomère et de segments de type monomère ou comonomère thermoplastique,
- (c) 0,1 à 10% en poids de particules creuses définies selon l'une quelconque des revendications 3 à 8, et,
- (d) 5 à 90% en poids d'au moins une huile synthétique.

10. Composition cosmétique déodorante anhydre comprenant :
- (a) 0,1 à 40% en poids d'au moins un actif déodorant,
- (b) 0,1 à 7% en poids d'au moins un copolymère di-, tri-, multi- ou radial bloc composé de segments de type styrène monomère et de segments de type monomère ou comonomère thermoplastique,
- (c) 0,1 à 5% en poids de particules creuses d'un terpolymère expansé de chlorure de vinylidène, d'acrylonitrile et de méthacrylate, et,
- (d) 5 à 90% en poids d'au moins une huile synthétique.

11. Composition selon la revendication 1, caractérisée par le fait que la substance absorbant le gras est choisie parmi le talc, l'amidon et ses dérivés, l'argile, la silice, les polyoléfines, le polystyrène, le téflon, et leurs mélanges.

12. Composition selon l'une quelconque des revendications précédentes, caractérisée par le fait que les huiles synthétiques sont choisies parmi les isoparaffines ou leurs mélanges de formule (I) suivante : 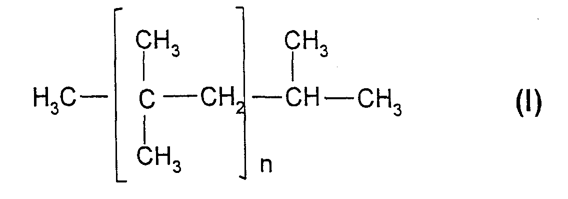 pour laquelle n ≥ 2 et de préférence compris entre 2 et 40.

13. Composition selon la revendication 12, caractérisée par le fait que l'huile synthétique est l'isohexadécane (n= 3).

14. Composition selon la revendication 12, caractérisée par le fait que l'huile synthétique est l'isododécane (n= 2).

15. Composition selon l'une quelconque des revendications précédentes, caractérisée par le fait que dans le copolymère bloc défini selon la revendication 1, les segments de type monomère ou comonomère thermoplastique, sont des segments éthylène/alkylène en C₃-C₄.

16. Composition selon la revendication 15, caractérisée par le fait que le copolymère bloc est un copolymère bloc hydrogéné à blocs styrène et à blocs éthylène/alkylène en C₃-C₄.

17. Composition selon la revendication 16, caractérisée par le fait que le copolymère bloc est constitué d'un mélange de copolymère hydrogéné à blocs butylène/éthylène et à blocs styrène et de copolymère hydrogéné à blocs éthylène/propylène et à blocs styrène dans de l'huile minérale.

18. Composition selon la revendication 17, caractérisée par le fait que le mélange de copolymère hydrogéné à blocs butylène/éthylène et à blocs styrène et de copolymère hydrogéné à blocs éthylène/propylène et à blocs styrène est présent à raison de 1 à 20% en poids dans 80 à 99% en poids d'huile minérale.

19. Composition selon l'une quelconque des revendications précédentes, caractérisée par le fait qu'elle contient en outre au moins un agent de consistance.

20. Composition selon la revendication 19, caractérisée par le fait l'agent de consistance est présent dans la composition dans une quantité suffisante pour obtenir un gel, une crème ou un stick.

21. Composition selon la revendication 19 ou 20, caractérisée par le fait l'agent de consistance est l'huile de ricin hydrogénée.

22. Composition selon l'une quelconque des revendications 1 à 18, caractérisée par le fait qu'elle contient en outre au moins un agent de suspension et qu'elle se présente sous forme d'aérosol.

23. Composition selon l'une quelconque des revendications précédentes, caractérisée par le fait que l'actif déodorant est choisi parmi les agents bactéricides et les absorbeurs d'odeurs.

24. Composition selon l'une quelconque des revendications 1 à 22, caractérisée par le fait que l'actif déodorant est un antitranspirant choisi parmi les sels d'aluminium, les sels de zirconium, les sels d'aluminium et de zirconium, les complexes d'hydroxychlorure de zirconium, d'hydroxychlorure d'aluminium avec la glycine.

25. Composition selon la revendication 24, caractérisée par le fait que l'actif antitranspirant est l'hydroxychlorure d'aluminium.

26. Utilisation cosmétique d'une composition selon l'une quelconque des revendications 1 à 25, pour réduire le flux sudoral et/ou masquer, améliorer ou réduire l'odeur désagréable résultant de la décomposition de la sueur humaine par des bactéries.

27. Procédé pour traiter les odeurs axillaires humaines, consistant à appliquer sur la surface axillaire une quantité efficace d'une composition selon l'une quelconque des revendications 1 à 26.
